(19) Europäisches Patentamt / European Patent Office / Office européen des brevets

(11) **EP 4 099 890 B1**

(12) **EUROPEAN PATENT SPECIFICATION**

(45) Date of publication and mention
of the grant of the patent:
**22.04.2026 Bulletin 2026/17**

(21) Application number: **20728238.5**

(22) Date of filing: **06.02.2020**

(51) International Patent Classification (IPC):
***A61B 5/00*** *(2006.01)*

(52) Cooperative Patent Classification (CPC):
**A61B 5/681; A61B 5/4812; A61B 5/6898;**
A61B 5/4815; A61B 5/4818

(86) International application number:
**PCT/RU2020/000059**

(87) International publication number:
**WO 2021/158137 (12.08.2021 Gazette 2021/32)**

(54) **SLEEP MONITORING USING PHOTOPLETHYSMOGRAPHY AND ACCELEROMETRY SENSORS IN COMBINATION WITH WI-FI CHANNEL STATE INFORMATION TECHNOLOGY**

SCHLAFÜBERWACHUNG MIT PHOTOPLETHYSMOGRAPHIE- UND BESCHLEUNIGUNGSSENSOREN IN KOMBINATION MIT WIFI-KANALZUSTANDSINFORMATIONSTECHNOLOGIE

SURVEILLANCE DU SOMMEIL À L'AIDE DE CAPTEURS DE PHOTOPLÉTHYSMOGRAPHIE ET D'ACCÉLÉROMÉTRIE EN COMBINAISON AVEC UNE TECHNOLOGIE D'INFORMATIONS D'ÉTAT DE CANAL WI-FI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**14.12.2022 Bulletin 2022/50**

(73) Proprietor: **Huawei Technologies Co., Ltd.**
**Shenzhen, Guangdong 518129, (CN)**

(72) Inventors:
• **OLESYUK, Roman Valeryevich**
  **Shenzhen Guangdong, 518129 (CN)**
• **BORISOV, Alexander Evgeyevich**
  **Shenzhen Guangdong, 518129 (CN)**
• **PIKHELETSKY, Mikhail Victorovich**
  **Shenzhen Guangdong, 518129 (CN)**
• **QU, Gaopeng**
  **Shenzhen Guangdong, 518129 (CN)**
• **QIU, Chen**
  **Shenzhen Guangdong, 518129 (CN)**

• **TATARAIDZE, Alexander Bidzinovich**
  **Shenzhen Guangdong, 518129 (CN)**

(74) Representative: **Thun, Clemens**
**Mitscherlich PartmbB**
**Karlstraße 7**
**80333 München (DE)**

(56) References cited:
**CN-A- 110 200 610     US-A1- 2018 199 882**

• **JIAN LIU ET AL: "Tracking Vital Signs During Sleep Leveraging Off-the-shelf WiFi", PROCEEDINGS OF THE 16TH ACM INTERNATIONAL SYMPOSIUM ON MOBILE AD HOC NETWORKING AND COMPUTING, MOBIHOC '15, 22 June 2015 (2015-06-22), New York, New York, USA, pages 267 - 276, XP055637026, ISBN: 978-1-4503-3489-1, DOI: 10.1145/2746285.2746303**

**Description**

BACKGROUND

The present disclosure relates to a system and method for monitoring sleep and, more specifically, but not exclusively, to a system for generating a recommendation for treating sleep disorders based on sleep-related features derived from data generated by two mobile devices.

Sleep consists of cycles. The length of each cycle is 90-120 minutes. A cycle consists of rapid-eye-movement (REM) sleep and non-REM sleep. According to the standard of the American Academy of Sleep Medicine (AASM), non-REM sleep is divided into three stages, NREM1, NREM2, and NREM3.

Insomnia and apnea are two common sleep disorders. Insomnia is difficulty falling asleep or staying asleep, and apnea is a disorder wherein breathing is interrupted during sleep. An apnea episode is a pause in breathing for 10 or more seconds with 3% or more of oxygen desaturation. Hypopnea refers to the decreasing of airflow by 30% or more, for 10 or more seconds.

Traditionally, sleep disorders have been diagnosed using polysomnography (PSG). PSG is a comprehensive recording of physiological changes during sleep. A typical setup for PSG requires attachment of numerous sensors to the body of the subject. PSG includes measurement of numerous physiological parameters during sleep, including, but not limited to: electroencephalogram (EEG) brain electrical activity; electrooculogram (EOG) - electrical activity of eye muscles; electromyogram (EMG) - electrical activity of skeletal muscles; electrocardiogram; airflow; thorax and abdominal respiratory movements; snoring; leg movements; photoplethysmography; posture, and video. Polysomnography is carried out in a sleep laboratory on certified equipment by a specialized physician, in accordance with the AASM manual. According to the AASM manual, a physician may visually analyze the EEG, EMG, and EOG signals to manually classify each epoch (defined as a 30 second interval) as wakefulness (W), REM (R), or the first to third stages of NREM sleep (N1-N3).

Polysomnography is used to calculate various sleep parameters and statistics. The sleep parameters include Total Recording Time (TRT) - time between going to bed and final wake up; Total Sleep Time (TST) - the Total Recording Time, excluding epochs of wakefulness; and Sleep Efficiency - percentage of Total Sleep Time compared to Total Recording Time. Other sleep parameters include Sleep Latency - the time from going to bed until the first sleep epoch; REM latency - time from going to bed until the first REM epoch; and N3 latency - time from going to sleep to the first N3 epoch. Additional sleep parameters include Wake after Sleep Onset (WASO), which is the total recording time minus the sum of sleep latency and total sleep time; the duration of sleep stages in minutes, and the duration of sleep stages as a percentage of the total sleep time.

Less intrusive devices for sleep monitoring have been developed, such as fitness trackers, smart watches, and smartphones. Sleep stages affect physiological signals that are detectable by such devices. For example, heart rate and respiratory pattern is quite variable during REM sleep and stable during N3 sleep. Motion occurs often during wakefulness, but is very rare during REM sleep. Apnea episodes also affect breathing patterns and heart rate. Accordingly, it is possible to use automatic analysis of heart rate variability and respiratory pattern for sleep monitoring.

One device for sleep monitoring is a smart wristband with photoplethysmography (PPG) and accelerometry (ACC) sensors. The sensor registers heart rate variability and motions.

Mobile phones may also be used for sleep monitoring. For example, mobile phones can analyze Wi-Fi channel state information and derive respiratory patterns therefrom. In addition, if a mobile phone is placed on a bed, it is possible to use an accelerometry sensor in the smartphone to register motions during sleep, for example, posture changes. It is also possible to use smartphones as a sonar for respiratory pattern monitoring, or as a sound recorder for recording of audio signals during sleep.

Further, US 2018/199882 A1 refers to a device and method for increasing a patient's compliance with a therapy relating to an upper airway disorder. The device comprises a receiving unit for receiving for receiving vital sign data including glucose level data of the patient, and therapy data including information regarding the therapy of the patient; a processing unit configured to process the therapy data and the vital sign data in order to determine feedback information based on the therapy data and the vital sign data; and a display unit for displaying the feedback information based on the therapy data and the vital sign data.

Further, the document JAIN LIU ET AL:" Tracking Vital Signs During Sleep Leveraging Off-the-shelf WiFi", Proceedings of the 16th ACM International Symposium on mobile Ad Hoc Networking and computing, June 22, 20155, New York, refers to a system reusing existing WiFi network and exploiting fine grained channel information to capture minute movements caused by breathing and heart beats. It describes breathing rate estimation of two persons in a bed with channel state information (CSI) measurements. The disclose system analyses the time series of CSI amplitude in frequency domain by using the power spectral density. The document explains that the person's chest or belly that is closer to the wireless link has bigger impact on the CSI changes, which creates more obvious periodic changes of CSI. This leads to the stronger peak corresponds to that persons breathing or heart beat rate. It is therefore possible to map the detected breathing or heart rates to each individual based on the strength of the peak and the proximity of the individual to the wireless link.

## SUMMARY

The accuracy of sensors currently used for sleep monitoring is limited. Non-contact sensors, such as radar, bed sensors, and smartphones, depend for their accuracy on various variables. Such variables include their location, environmental characteristics (such as softness of a mattress), presence or absence of another moving object (such as a sleep partner, or a pet), position of the subject, and other similar variables. For smart wristbands, the quality of signals is dependent on strap tension. Moreover, the amount of sensors on a wristband and their characteristics, such as the sampling frequency, are limited by the small size and battery life of the wristband. As a result, these devices are limited in their accuracy.

It is an object of the present invention to provide a system for generating a recommendation for generating sleep disorders, using one or more of the above-referenced devices, with increased accuracy.

The foregoing and other objects are achieved by the features of the independent claim.

Further implementation forms are apparent from the dependent claims, the description and the figures.

In a first aspect a system for generating recommendations for treating sleep disorders is provided, the system comprising a plurality of first mobile devices configured to be used by a plurality of patients, a single second mobile device, and a Wi-Fi access point,

○ wherein each of the plurality of first mobile devices comprises a photoplethysmography sensor, an accelerometry sensor, and a communications module, and each first mobile device of the plurality of first mobile devices is associated with a respective different patient of the plurality of patients during use,
○ wherein the single second mobile device comprises a Wi-Fi transceiver for receiving Wi-Fi packets from the Wi-Fi access point and a processing circuitry, and the single second mobile device is associated with all of the plurality of patients during use, wherein:

• the processing circuitry of the second mobile device is adapted to, for each patient among the plurality of patients, execute a code for:

○ deriving a first preliminary set of sleep-related features based on photoplethysmography and accelerometry data collected by the first mobile device among the plurality of first mobile devices to which the patient is associated to;
○ identifying Wi-Fi channel state information associated with an activity of the patient by correlating Wi-Fi channel state information collected by the single second mobile device for all patients among the plurality of patients with the photoplethysmography or accelerometry data gathered by said patient's first mobile device;
○ deriving a second preliminary set of sleep-related features based on the identified Wi-Fi channel state information associated with the activity of the patient;
○ generating a combined set of sleep-related features of the patient by combining the first preliminary set of sleep-related features of the patient with the second preliminary set of sleep-related features of the patient, and
○ providing a recommendation for treating a sleep disorder of the patient based on the combined set of sleep-related features of the patient.

An advantage of the system is that multiple sensors may be used to measure the same parameters and thereby determine the same sleep-related features. This allows for increasing the accuracy of the calculation of these parameters, and as a result the accuracy of the sleep monitoring as a whole. For example, for determination of a respiratory pattern, data from both photoplethysmography sensors on a wristband and Wi-Fi channel state information on a mobile phone may be collected and analyzed. The combination of such data may provide more accurate information about a respiratory pattern than either of the sensors on its own.

In another implementation according to the first aspect, at least some sleep-related features from the first preliminary set are not present in the second preliminary set, or vice versa, and the processing circuitry in the second mobile device is adapted to calculate the combined set of sleep-related features by concatenating different sleep-related features from the first preliminary set and the second preliminary set.

Advantageously, the simultaneous use of two different sensing devices allows for the collection of more parameters compared to what can be collected from just one of them. For example, a light sensor and microphone on a mobile phone may be used for estimation of the suitability of the environment for sleep, based on illumination and noise level, without a parallel measurement with a wristband sensor. The illumination and noise level measurements may be useful for sleep staging and estimation of circadian rhythms. Thus, the processing circuitry derives a concatenated list of sleep-related features based on inputs from both mobile devices.

In another implementation according to the first aspect, at least some sleep-related features from the first preliminary set are present in the second preliminary set, and the processing circuitry in the second mobile device is, for generating the combined set of sleep-related features of the patient, adapted, for each feature of the patient that is present in both preliminary sets, to calculate a combined value for that respective feature as a function of a value for that respective feature of the patient in each of the first and second preliminary sets.

Advantageously, the combined value may thus be more accurate than the analogous value that is derived from the first preliminary set or the second preliminary set alone.

Other systems, methods, features, and advantages of the present disclosure will be or become apparent to one with skill in the art upon examination of the following drawings and detailed description.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 is a schematic diagram illustrating a system for generating a recommendation for treating sleep disorders in a single patient, according to embodiments of the invention;
FIG. 2 is a depiction of steps of deriving a sleep related feature based on data collected by a first mobile device or a second mobile device, according to embodiments of the invention;
FIG. 3 is a depiction of sleep staging data generated by combining data from a first mobile device and a second mobile device, according to embodiments of the invention; and
FIG. 4 is a schematic diagram illustrating the system of FIG. 1 monitoring sleep of multiple patients in a single room, according to embodiments of the invention.

DETAILED DESCRIPTION

The present disclosure relates to a system (part of the present invention) and a method (just an example not part of the present invention) for monitoring sleep and, more specifically, but not exclusively, to a system for generating a recommendation for treating sleep disorders based on sleep-related features derived from data generated by two mobile devices.

Before explaining at least one embodiment of the invention in detail, it is to be understood that the disclosure is not necessarily limited in its application to the details of construction and the arrangement of the components and/or methods set forth in the following description and/or illustrated in the drawings and/or the Examples. The invention is capable of other embodiments or of being practiced or carried out in various ways.

A computer program product may include a computer readable storage medium (or media) having computer readable program instructions, wherein, when the computer readable instructions are executed by a processor, the processor is caused to perform the disclosed methods.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing.

Computer readable program instructions described herein can be downloaded to respective computing/processing devices from a computer readable storage medium or to an external computer or external storage device via a network, for example, the Internet, a local area network, a wide area network and/or a wireless network.

The computer readable program instructions may be executed entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

The system, method, and device described herein includes processing circuitry configured to perform, conduct or initiate various operations of the device described in the following. As used in the present disclosure, the term "processing circuitry" may comprise hardware and software. The hardware may comprise analog circuitry or digital circuitry, or both analog and digital circuitry. The digital circuitry may comprise components such as application-specific integrated circuits (ASICs), field-programmable arrays (FPGAs), digital signal processors (DSPs), or multipurpose processors. In one embodiment, the processing circuitry comprises one or more processors and a non-transitory memory connected to the one or more processors. The non-transitory memory may carry executable program code which, when executed by the one or more processors, causes the device to perform, conduct or initiate the operations or methods described below.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

The flowchart and block diagrams in the Figures illustrate the architecture, functionality, and operation of possible implementations of systems, methods, and computer program products according to this disclosure. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

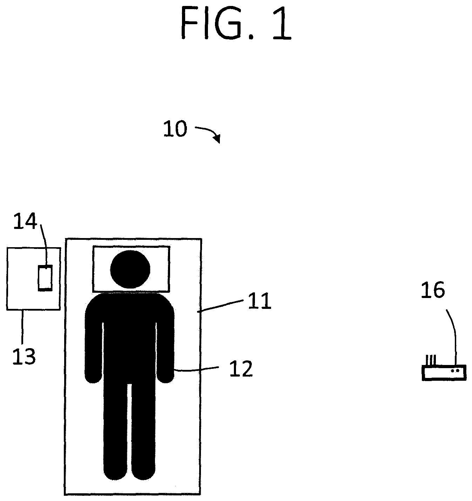

Reference is now made to FIG. 1, which is a schematic depiction of a system 10 for sleep monitoring. System 10 includes a first mobile device 12, which, in the depicted embodiment, is a wristband tracker. System 10 also includes a second mobile device 14, which, in the depicted embodiment, is a mobile phone. The second mobile device 14 may be any other mobile device with the functionalities depicted herein. For example, the second mobile device 14 may be a smart speaker, which in currently available commercial embodiments includes both an ultrasound sensor and a Wi-Fi transceiver. As used in the present disclosure, the terms "first mobile device" and "wristband tracker," and the terms "second mobile device" and "mobile phone," are used interchangeably.

The system 10 is placed in a location within range of a Wi-Fi access point 16. In the illustrated embodiment, the patient is reclining on bed 11, wearing the wristband tracker 12, and the mobile phone 14 is placed on a bedside table 13 adjacent to the patient's head.

First mobile device 12 is equipped with photoplethysmography (PPG) and accelerometry (ACC) sensors, which function in the manner of such sensors that are known to those of skill in the art. In addition to the PPG and ACC sensors, the wristband tracker 12 may include one or more additional sensors. These additional sensors may include a lighting sensor, video camera, time of flight camera, gyroscope, microscope, temperature sensor, piezoelectric sensor, or galvanic skin sensor. The mobile device 12 collects and records data acquired by the various sensors.

First mobile device 12 also includes a communications module, for example, a Bluetooth or a Wi-Fi transceiver, which may be used to transfer data between the first mobile device 12 and the second mobile device 14.

First mobile device 12 also includes processing circuitry. The processing circuitry is configured to execute a code to derive a first preliminary set of sleep-related features based on the data collected from the sensors, such as the PPG sensor and the ACC sensor. The sleep-related features include features related to at least one of breathing, heartbeat, and gross body motions.

Second mobile device 14 includes a Wi-Fi transceiver that receives Wi-Fi packets from Wi-Fi access point 16. The second mobile device 14 contains processing circuitry for recording Wi-Fi channel state information (CSI). CSI represents how wireless signals propagate from a transmitter to a receiver at certain carrier frequencies along multiple paths. CSI is a 3D matrix of complex values representing the amplitude attenuation and phase shift of multi-path Wi-Fi channels. A time series of CSI measurements captures how wireless signals travel through surrounding objects and humans in time, frequency, and spatial domains. CSI tracking may be used for sleep monitoring. For example, CSI may be used to estimate breathing rate, because phase shifts in the time domain may have different dominant frequency components due to the patient's breathing.

The processing circuitry of the mobile phone 14 is adapted to execute a code for deriving a second preliminary set of sleep-related features. These features are based on the wireless network channel state information collected by the mobile phone 14. This second preliminary set of sleep-related features may, for example, include features related to breathing, heartbeat, and gross-body motions.

The mobile phone 14 may also include additional sensors, beyond a Wi-Fi transceiver. For example, the mobile phone 14 may include an audio signal recorder, which records an audio signal. The processing circuitry of the mobile phone may be further adapted to extract data from the recorded audio signal and to derive sleep-related features from the audio signal data. The mobile phone 14 may also include a speaker for transmitting sound signals. The speaker and the audio signal recorder may function together, to enable performance of sonar calculations based on frequency shifts caused by breathing movements. The wristband tracker 12 or the mobile phone 14 may also send frequency modulated continuous wave transmissions, and either the wristband tracker 12 or the mobile phone 14 may also receive reflected signals (*e.g.*, signals reflected off of the patient). The wave transmissions may alternatively be modulated in their pulse, phase, or amplitude. The processing circuitry of the mobile phone is adapted to derive sleep-related data, for example, respiratory information, from the sonar transmission.

The foregoing examples of sensors in the first mobile device 12 and the second mobile device 14 are not meant to be exhaustive. The first and second mobile devices 12, 14 may incorporate any sensor that is currently known or that later will become known. In addition, aside from the PPG and ACC sensors on the first mobile device 12, and the Wi-Fi CSI detector on the second mobile device 14, any of the above-referenced additional sensors may be located on either the first mobile device 12, or the second mobile device 14, or may exist in duplicate on both mobile devices. Also, the first and second preliminary set of sleep-related features may include any other physiological or environmental features relevant to analysis of sleep, and are not limited to breathing, heartbeat, and gross body motions.

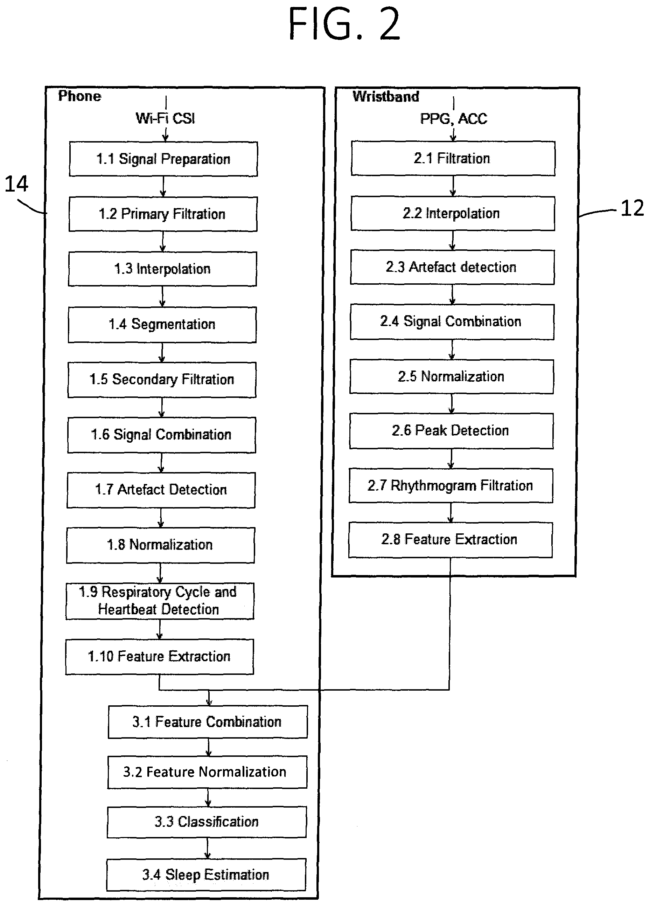

FIG. 2 depicts a flow chart for the derivation of the sleep-related features from the raw data gathered by the first mobile device and second mobile device.

As seen on the left side of FIG. 2, after the mobile phone 14 receives Wi-Fi packets from Wi-Fi access point 16, the processing circuitry of the mobile phone 14 receives Wi-Fi CSI raw data from the Wi-Fi transceiver. As discussed above, the mobile phone 14 may also receive data from other sensors. The processing circuitry then engages in the following steps: signal preparation (step 1.1); primary filtration (step 1.2); interpolation (step 1.3); segmentation (step 1.4); secondary filtration (step 1.5); signal combination (step 1.6); artefact detection (step 1.7); normalization (step 1.8); respiratory cycle and heartbeat detection (step 1.9); and feature extraction (step 1.10). These steps may be performed in the manner known to those of skill in the art. The second preliminary set of sleep-related features is an output of the feature extraction step 1.10. As discussed above, these features may include features related to breathing, heartbeats, and gross body motions.

As seen on the right side of FIG. 2, the processing circuitry of the wristband 12 receives raw data from the PPG and ACC sensors. As discussed above, the wristband 12 may also receive data from other sensors. The processing circuitry jointly analyzes the PPG and ACC signals, in the following steps: filtration (step 2.1); interpolation (step 2.2); artefact detection (step 2.3); signal combination (step 2.4); normalization (step 2.5); peak detection (step 2.6); rhythmogram filtration (step 2.7); and feature extraction (step 2.8). These steps may also be performed in the manner known to those of skill in the art. The first preliminary set of sleep-related features is an output of the feature extraction step 2.8. These features, like the features derived from the Wi-Fi CSI, may include features related to breathing, heartbeats, and gross body motions.

The first preliminary set of features is then transmitted wirelessly to the mobile phone 14, for example, through a Bluetooth or Wi-Fi connection. This transmission may take place continuously (e.g., every N seconds), or may take place in a single transmission, at the conclusion of a sleep session.

In an alternative embodiment, instead of transmitting the derived sleep-related features, the first mobile device 12 may send raw data collected from its sensors (e.g., the PPG and ACC sensors) to the second mobile device 14 via the wireless connection. The processing circuitry of the second mobile device 14 then derives the first preliminary set of sleep-related features, in addition to deriving the second preliminary set of sleep-related features. This alternative arrangement may be desirable, for example, when the second mobile device 14 has greater computing power or is easier to program.

After the transmission of the sets of features from the wristband 12 to the mobile phone 14, at step 3.1, the processing circuitry of the mobile phone then combines the features into a single set. As part of this process, a combined set of sleep-related features is generated, by evaluating the sleep-related features from one of the first or second preliminary steps, and identifying analogous sleep-related features from the other one of the first or second preliminary sets.

The features from mobile phone 14 and a wristband tracker 12 are combined in two ways. When features are different, the two feature sets may simply be concatenated into the final one. When the features are derived from measurements taken by both devices (e.g. respiratory rate estimated by the wristband 12 and the phone 14 independently), a function is used to produce the final feature based on the two features:

$$x_{final}^1 = f\big(x_{phone}^1, x_{wristband}^1\big)$$

For example, the final calculated value could be a weighted average taken from both preliminary values, according to the equation:

$$x_{final}^1 = w_1 x_{phone}^1 + w_2 x_{wristband}^1.$$

At step 3.2, the processing circuitry performs feature normalization. At this stage, the concatenated data set is normalized onto a single scale. Then, at step 3.3, the processing circuitry performs classification. For example, the data is classified into specific patterns.

Finally, at step 3.4, the processing circuitry conducts a sleep estimation, in which it estimates the duration of each of the patient's sleep cycles during a sleep measurement episode. In particular, during step 3.4, a machine-learning algorithm based on the combined feature set classifies each thirty-second epoch to one of the following sleep stages: W, R, light sleep (N1&N2), or deep sleep (N3). A machine-learning algorithm based on the combined feature set also classifies each epoch as apneic or normal. Sleep parameters, Apnea-Hypopnea Index (AHI), and sleep quality are calculated based on the results of classification. Probability of sleep disorders are calculated based on the analysis of these metrics.

The processing circuitry may also determine a pattern of the patient's sleep stages. For example, the pattern may be: (a) wake, sleep; (b) wake, REM sleep, NREM sleep; (c) wake, REM sleep, light sleep, deep sleep; or (d) wake, REM, NREM1, NREM2, NREM3.

The processing circuitry is also adapted to calculate sleep statistics associated with the patient. The sleep statistics may be selected from a group consisting of: total recording time, total sleep time, sleep period, lights out and lights on times, sleep efficiency, sleep quality, sleep latency, REM latency, number of awakenings, wake after sleep onset, and duration of sleep stages.

The processing circuitry is also adapted to execute code to provide a recommendation for treating a sleep disorder based on the combined set of sleep-related features. The recommendation may include, for example, taking medication before sleep; using a breathing machine during sleep; using a dental guard during sleep; changing sleep posture; or changing sleep environment.

As discussed above, at least some sleep-related features from the first preliminary set are present in the second preliminary set. The processing circuitry is adapted, for each such feature that is present in both preliminary sets, to calculate a combined value for that respective feature as a function of a value for that respective feature in each of the first and second preliminary sets. For example, for determination of a respiratory pattern, data from both photoplethysmography sensors on a wristband 12 and Wi-Fi channel state information on a mobile phone 14 may be collected and analyzed. The combination of such data may provide more accurate information about a respiratory pattern than either of the sensors on its own.

In an additional embodiment, at least some sleep-related features from the first preliminary set are not present in the second preliminary set, or vice versa, and the processing circuitry is adapted to calculate a combined set of sleep-related features by concatenating different sleep-related features from the first preliminary set and from the second-preliminary set. For example, a light sensor and microphone on a mobile phone 14 may be used for estimation of the suitability of the environment for sleep, based on illumination and noise level, without a parallel measurement on the wristband tracker 12. The illumination and noise level measurements may be useful for sleep staging and estimation of circadian rhythms. Thus, the processing circuitry derives a concatenated list of sleep-related features based on inputs from both mobile devices.

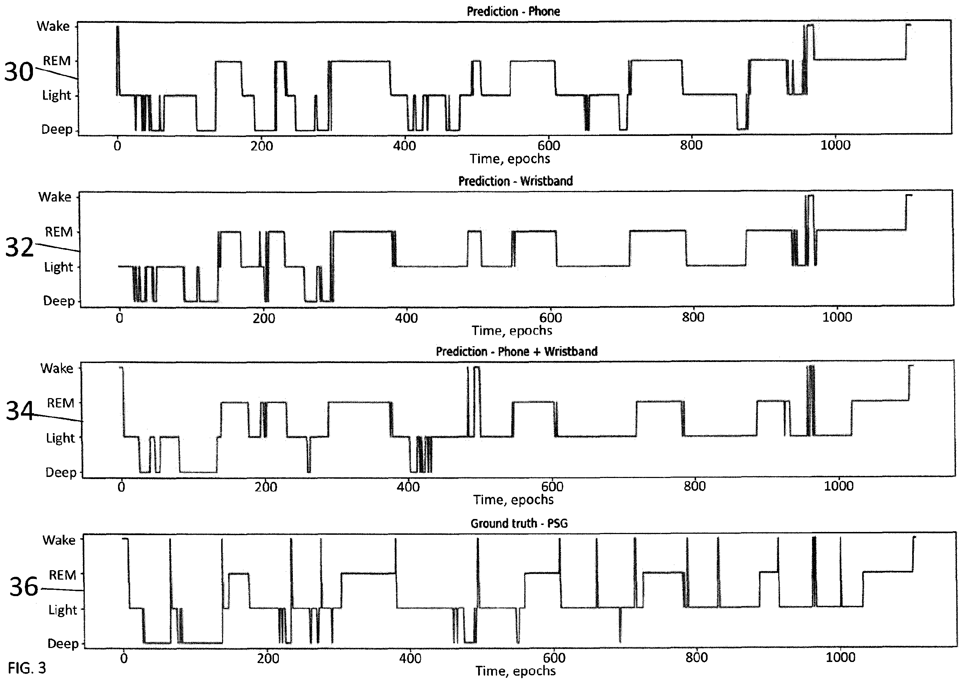

FIG. 3 depicts sleep staging data for a single patient using various techniques, and illustrates the increased accuracy achieved by the combination of data from the first and second mobile devices. In FIG. 3, chart 30 depicts stages of sleep in a patient, as calculated based on Wi-Fi channel state information data collected by a mobile phone. Chart 32 depicts the stages of sleep in the same patient, as calculated based on ACC and PSG data collected by a wristband tracker. Chart 34 depicts the sleep stages of the same patient, as calculated by combination of the data collected by the mobile phone and wristband tracker, in the manner described above. Finally, chart 36 depicts the sleep stages of the same patient, as calculated based on polysomnography data. As can be seen, the prediction of sleep stages in chart 34 more closely matches the sleep stages determined in chart 36 than either of the predicted sleep stages of charts 30 or 32.

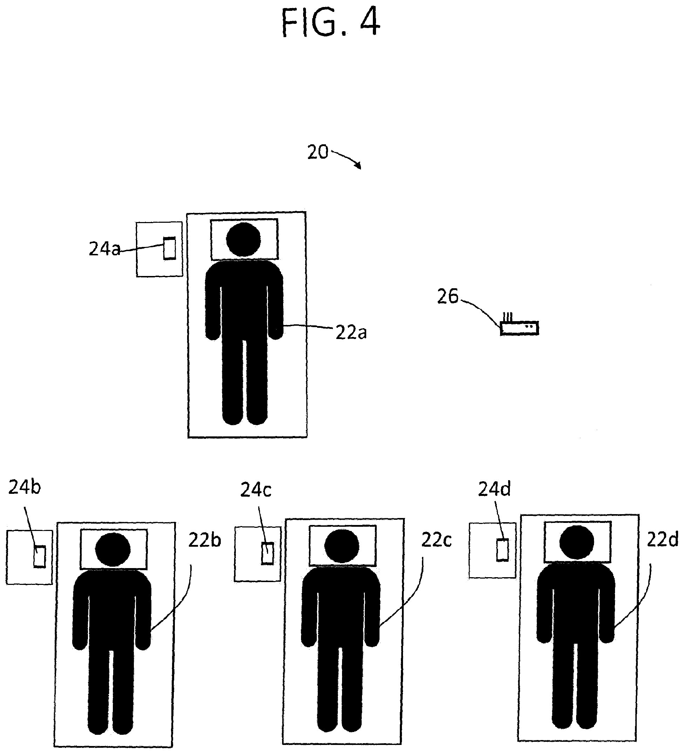

FIG. 4 depicts a system 20 for generating a recommendation for treating sleep disorders in multiple patients sleeping in the same room. The system 20 of FIG. 4 is similar to that of FIG. 1, and accordingly similar reference numerals are used, except that elements are identified with reference numerals beginning with "2" rather than "1." In the illustrated embodiment, there are four patients; however, there could be fewer or more than four patients as well. As seen in FIG. 4, for each of the patients, the system includes a wristband tracker 22a, 22b, 22c, or 22d. In the depicted embodiment, there is also a single mobile phone 24a, 24b, 24c, 24d associated with each patient. According to the present invention, a single mobile phone, e.g., mobile phone 24a, is associated with all of the patients. A single Wi-Fi transceiver 26 serves all of the patients.

In operation, each of the wristband trackers 22a-d monitors an associated patient with photoplethysmography and accelerometry sensors. The ACC and PPG sensor data is used to derive preliminary sets of sleep-related features, such as features related to respiration, as discussed above. Either each of the mobile phones 24a-d, or a single mobile phone 24a monitors Wi-Fi channel state information based on movements of all of the patients. In the embodiment according to the invention, in which only mobile phone 24a monitors the Wi-Fi channel state information, without additional information, it would not be possible to determine which patient's respiration corresponds to changes in the CSI pattern. To provide this information, the respiratory features obtained from each of the wristband trackers 22a-d are correlated to the respiratory features obtained by the mobile phone 24a. Information about each patient's respiratory rate, derived from the ACC and PPG data collected by the wristband trackers 22a-d, is used to determine which respiratory patterns from the Wi-Fi CSI result from each patient. Advantageously, the system 20 may be used to efficiently and unobtrusively monitor the sleep of a plurality of individuals sleeping in the same room, *e.g.*, a married couple or siblings.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

**Claims**

1. A system (20) for generating recommendations for treating sleep disorders, the system comprising a plurality of first mobile devices (22a, 22b, 22c, 22d) configured to be used by a plurality of patients, a single second mobile device (24a), and a Wi-Fi access point (26),

   ◦ wherein each of the plurality of first mobile devices (22a, 22b, 22c, 22d) comprises a photoplethysmography sensor, an accelerometry sensor, and a communications module, and each first mobile device of the plurality of first mobile devices (22a, 22b, 22c, 22d) is associated with a respective different patient of the plurality of patients during use,
   ◦ wherein the single second mobile device (24a) comprises a Wi-Fi transceiver for receiving Wi-Fi packets from the Wi-Fi access point and a processing circuitry, and the single second mobile device (24a) is associated with all of the plurality of patients during use,

   wherein:

   • the processing circuitry of the second mobile device (24a) is adapted to, for each patient among the plurality of patients, execute a code for:

      ◦ deriving a first preliminary set of sleep-related features based on photoplethysmography and accelerometry data collected by the first mobile device (22a, 22b, 22c, 22d) among the plurality of first mobile devices (22a, 22b, 22c, 22d) to which the patient is associated to;
      ◦ identifying Wi-Fi channel state information associated with an activity of the patient by correlating Wi-Fi channel state information collected by the single second mobile device (24a) for all patients among the plurality of patients with the photoplethysmography or accelerometry data gathered by said patient's first mobile device (22a, 22b, 22c, 22d);
      ◦ deriving a second preliminary set of sleep-related features based on the identified Wi-Fi channel state information associated with the activity of the patient;
      ◦ generating a combined set of sleep-related features of the patient by combining the first preliminary set of sleep-related features of the patient with the second preliminary set of sleep-related features of the patient, and
      ◦ providing a recommendation for treating a sleep disorder of the patient based on the combined set of sleep-related features of the patient.

2. The system (20) of claim 1, wherein at least some sleep-related features from the first preliminary set are not present in the second preliminary set, or vice versa, and the processing circuitry in the second mobile device (24a) is adapted to calculate the combined set of sleep-related features by concatenating different sleep-related features of the patient from the first preliminary set and from the second-preliminary set.

3. The system (20) of claim 1, wherein at least some sleep-related features from the first preliminary set are present in the second preliminary set, and the processing circuitry in the second mobile device (24a) is, for generating the combined set of sleep-related features of the patient, adapted, for each feature of the patient that is present in both preliminary sets, to calculate a combined value for that respective feature as a function of a value for that respective feature of the patient in each of the first and second preliminary sets.

**Patentansprüche**

1. System (20) zum Generieren von Empfehlungen zum Behandeln von Schlafstörungen, wobei das System eine Vielzahl von ersten mobilen Vorrichtungen (22a, 22b, 22c, 22d), die dazu konfiguriert ist, durch eine Vielzahl von Patienten verwendet zu werden, eine einzelne zweite mobile Vorrichtung (24a) und einen Wi-Fi-Zugangspunkt (26) umfasst,

   ◦ wobei jede der Vielzahl von ersten mobilen Vorrichtungen (22a, 22b, 22c, 22d) einen Photoplethysmographiesensor, einen Beschleunigungssensor und ein Kommunikationsmodul umfasst, und jede erste mobile Vorrichtung der Vielzahl von ersten mobilen Vorrichtungen (22a, 22b, 22c, 22d) bei der Verwendung mit einem jeweiligen anderen Patienten der Vielzahl von Patienten assoziiert ist,

∘ wobei die einzelne zweite mobile Vorrichtung (24a) einen Wi-Fi-Sende-Empfänger zum Empfangen von Wi-Fi-Paketen von dem Wi-Fi-Zugangspunkt und eine Verarbeitungsschaltung umfasst, und die einzelne zweite mobile Vorrichtung (24a) bei der Verwendung mit allen der Vielzahl von Patienten assoziiert ist, wobei:

• die Verarbeitungsschaltung der zweiten mobilen Vorrichtung (24a) geeignet ist, um für jeden Patienten unter der Vielzahl von Patienten einen Code für Folgendes auszuführen:

∘ Ableiten eines ersten vorläufigen Satzes von schlafbezogenen Merkmalen basierend auf Photoplethysmographie- und Beschleunigungsdaten, die durch die erste mobile Vorrichtung (22a, 22b, 22c, 22d) unter der Vielzahl von ersten mobilen Vorrichtungen (22a, 22b, 22c, 22d), mit welchen der Patient assoziiert ist, erfasst werden;
∘ Identifizieren von Wi-Fi-Kanalzustandsinformationen, die mit einer Aktivität des Patienten assoziiert sind, durch Korrelieren der Wi-Fi-Kanalzustandsinformationen, die durch die zweite mobile Vorrichtung (24a) für alle Patienten unter der Vielzahl von Patienten erfasst werden, mit den Photoplethysmographie- oder Beschleunigungsdaten, die durch die erste mobile Vorrichtung (22a, 22b, 22c, 22d) des Patienten gesammelt werden; ∘ Ableiten eines zweiten vorläufigen Satzes von schlafbezogenen Merkmalen basierend auf den identifizierten Wi-Fi-Kanalzustandsinformationen, die mit der Aktivität des Patienten assoziiert sind;
∘ Generieren eines kombinierten Satzes von schlafbezogenen Merkmalen des Patienten durch Kombinieren des ersten vorläufigen Satzes von schlafbezogenen Merkmalen des Patienten mit dem zweiten vorläufigen Satz von schlafbezogenen Merkmalen des Patienten, und
∘ Bereitstellen einer Empfehlung zum Behandeln einer Schlafstörung des Patienten basierend auf dem kombinierten Satz von schlafbezogenen Merkmalen des Patienten.

**2.** System (20) nach Anspruch 1, wobei mindestens einige schlafbezogene Merkmale aus dem ersten vorläufigen Satz nicht in dem zweiten vorläufigen Satz vorhanden sind oder umgekehrt, und die Verarbeitungsschaltung in der zweiten mobilen Vorrichtung (24a) geeignet ist, um den kombinierten Satz von schlafbezogenen Merkmalen durch Verketten verschiedener schlafbezogener Merkmale des Patienten aus dem ersten vorläufigen Satz und aus dem zweiten vorläufigen Satz zu berechnen.

**3.** System (20) nach Anspruch 1, wobei mindestens einige schlafbezogene Merkmale aus dem ersten vorläufigen Satz in dem zweiten vorläufigen Satz vorhanden sind, und die Verarbeitungsschaltung in der zweiten mobilen Vorrichtung (24a) zum Generieren des kombinierten Satzes von schlafbezogenen Merkmalen des Patienten geeignet ist, um für jedes Merkmal des Patienten, das in beiden vorläufigen Sätzen vorhanden ist, einen kombinierten Wert für dieses jeweilige Merkmal als eine Funktion eines Wertes für dieses jeweilige Merkmal des Patienten in jedem des ersten und des zweiten vorläufigen Satzes zu berechnen.

**Revendications**

**1.** Système (20) de génération de recommandations pour le traitement de troubles du sommeil, le système comprenant une pluralité de premiers dispositifs mobiles (22a, 22b, 22c, 22d) configurés pour être utilisés par une pluralité de patients, un second dispositif mobile unique (24a), et un point d'accès Wi-Fi (26),

∘ dans lequel chacun de la pluralité de premiers dispositifs mobiles (22a, 22b, 22c, 22d) comprend un capteur de photopléthysmographie, un capteur d'accélérométrie, et un module de communication, et chaque premier dispositif mobile de la pluralité de premiers dispositifs mobiles (22a, 22b, 22c, 22d) est associé à un patient différent de la pluralité de patients pendant son utilisation,
∘ dans lequel le second dispositif mobile unique (24a) comprend un émetteur-récepteur Wi-Fi destiné à recevoir des paquets Wi-Fi à partir du point d'accès Wi-Fi et un circuit de traitement, et le second dispositif mobile unique (24a) est associé à l'ensemble de la pluralité de patients pendant son utilisation, dans lequel :

• le circuit de traitement du second dispositif mobile (24a) est adapté pour, pour chaque patient parmi la pluralité de patients, exécuter un code pour :

        ∘ dériver un premier ensemble préliminaire de caractéristiques liées au sommeil sur la base de données de photopléthysmographie et d'accélérométrie collectées par le premier dispositif mobile (22a, 22b, 22c, 22d) parmi la pluralité de premiers dispositifs mobiles (22a, 22b, 22c, 22d) auxquels le patient est associé ; o identifier des informations d'état de canal Wi-Fi associées à une activité du patient en corrélant les informations d'état de canal Wi-Fi collectées par le second dispositif mobile unique (24a) pour tous les patients parmi la pluralité de patients avec les données de photopléthysmographie ou d'accélérométrie recueillies par ledit premier dispositif mobile dudit patient (22a, 22b, 22c, 22d) ;

        ∘ dériver un second ensemble préliminaire de caractéristiques liées au sommeil sur la base des informations d'état de canal Wi-Fi identifiées associées à l'activité du patient ;

        ∘ générer un ensemble combiné de caractéristiques liées au sommeil du patient en combinant le premier ensemble préliminaire de caractéristiques liées au sommeil du patient avec le second ensemble préliminaire de caractéristiques liées au sommeil du patient, et

        ∘ fournir une recommandation pour le traitement d'un trouble du sommeil du patient sur la base de l'ensemble combiné des caractéristiques liées au sommeil du patient.

**2.** Système (20) selon la revendication 1, dans lequel au moins certaines caractéristiques liées au sommeil provenant du premier ensemble préliminaire ne sont pas présentes dans le second ensemble préliminaire, ou vice versa, et le circuit de traitement dans le second dispositif mobile (24a) est adapté pour calculer l'ensemble combiné de caractéristiques liées au sommeil en concaténant différentes caractéristiques liées au sommeil du patient provenant du premier ensemble préliminaire et du second ensemble préliminaire.

**3.** Système (20) selon la revendication 1, dans lequel au moins certaines caractéristiques liées au sommeil provenant du premier ensemble préliminaire sont présentes dans le second ensemble préliminaire, et le circuit de traitement du second dispositif mobile (24a) est, pour générer l'ensemble combiné de caractéristiques liées au sommeil du patient, adapté, pour chaque caractéristique du patient qui est présente dans les deux ensembles préliminaires, pour calculer une valeur combinée pour cette caractéristique respective en fonction d'une valeur pour cette caractéristique respective du patient dans chacun des premier et second ensembles préliminaires.

# FIG. 1

# FIG. 2

**Phone**

Wi-Fi CSI

14

| 1.1 Signal Preparation |
| 1.2 Primary Filtration |
| 1.3 Interpolation |
| 1.4 Segmentation |
| 1.5 Secondary Filtration |
| 1.6 Signal Combination |
| 1.7 Artefact Detection |
| 1.8 Normalization |
| 1.9 Respiratory Cycle and Heartbeat Detection |
| 1.10 Feature Extraction |

**Wristband**

PPG, ACC

12

| 2.1 Filtration |
| 2.2 Interpolation |
| 2.3 Artefact detection |
| 2.4 Signal Combination |
| 2.5 Normalization |
| 2.6 Peak Detection |
| 2.7 Rhythmogram Filtration |
| 2.8 Feature Extraction |

| 3.1 Feature Combination |
| 3.2 Feature Normalization |
| 3.3 Classification |
| 3.4 Sleep Estimation |

FIG. 3

# FIG. 4

## REFERENCES CITED IN THE DESCRIPTION

**Patent documents cited in the description**

- US 2018199882 A1

**Non-patent literature cited in the description**

- **JAIN LIU et al.** Tracking Vital Signs During Sleep Leveraging Off-the-shelf WiFi. *Proceedings of the 16th ACM International Symposium on mobile Ad Hoc Networking and computing*